(19) Europäisches Patentamt
European Patent Office
Office européen des brevets

(11) Numéro de publication : **0 127 536**
**B1**

(12) **FASCICULE DE BREVET EUROPÉEN**

(45) Date de publication du fascicule du brevet : **21.01.87**

(21) Numéro de dépôt : **84401052.0**

(22) Date de dépôt : **22.05.84**

(51) Int. Cl.[4] : **C 07 C 45/51**, C 07 C 49/203, C 07 C 49/587, C 07 C 47/21, C 07 C 45/62, C 07 C 49/04

(54) **Procédé de préparation de composés carbonyles alpha-delta-diéthyléniques.**

(30) Priorité : **27.05.83 FR 8308809**

(43) Date de publication de la demande : **05.12.84 Bulletin 84/49**

(45) Mention de la délivrance du brevet : **21.01.87 Bulletin 87/04**

(84) Etats contractants désignés : **AT BE CH DE FR GB IT LI LU NL SE**

(56) Documents cités :
**EP-A- 0 069 010**
**CH-A- 521 298**
**FR-A- 1 576 228**
**FR-A- 2 072 545**
**FR-A- 2 340 923**
**NL-A- 64 579**
**COLLECTION OF CZECHOSLOVAK CHEMICAL COMMUNICATIONS, vol. 37, 1972, pages 461-465; M. KRAUS: "Palladium-catalyzed isomerization of unsaturated alcohols"**
**Le dossier contient des informations techniques présentées postérieurement au dépôt de la demande et ne figurant pas dans le présent fascicule.**

(73) Titulaire : **RHONE-POULENC SANTE
Les Miroirs 18 Avenue d'Alsace
F-92400 Courbevoie Cedex (FR)**

(72) Inventeur : **Bluthe, Norbert
39, rue Clément Michut
F-69100 Villeurbanne (FR)**
Inventeur : **Falou, Armand Samir
24, rue Boileau
F-69006 Lyon (FR)**
Inventeur : **Gore, Jacques
7, rue du Mont-Cindre
F-69300 Caluire (FR)**

(74) Mandataire : **Pilard, Jacques et al
RHONE-POULENC RECHERCHES Service Brevets Pharma 25, Quai Paul Doumer
F-92408 Courbevoie Cedex (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne un procédé de préparation de composés carbonylés α, δ-diéthyléniques de formule générale :

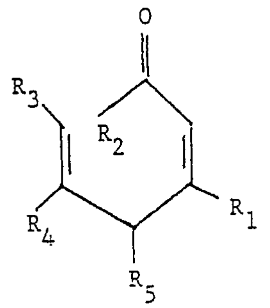

(I)

dans laquelle $R_1$, $R_2$, $R_3$ et $R_4$, identiques ou différents, représentent un atome d'hydrogène ou un radical hydrocarboné, $R_5$ représente un atome d'hydrogène, étant entendu que $R_2$ et $R_3$ peuvent former ensemble un radical alcoylène $(-CH_2-)_n$ dont un ou plusieurs atomes de carbone peuvent être éventuellement substitués par un ou plusieurs radicaux alcoyles contenant 1 à 4 atomes de carbone et dans lequel n représente un nombre entier compris entre 3 et 30 inclusivement et que $R_2$ et $R_5$ peuvent former ensemble un cycle contenant de 6 à 16 chaînons, par transposition d'un alcool acétylénique de formule générale :

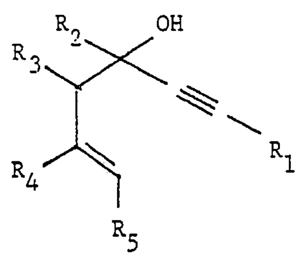

(II)

dans laquelle $R_1$, $R_2$, $R_3$, $R_4$ et $R_5$ sont définis comme précédemment.

Par radical hydrocarboné est entendu un radical acyclique contenant 1 à 20 atomes de carbone, dont la chaîne peut contenir une ou plusieurs doubles ou triples liaisons.

D'un intérêt tout particulier sont les produits de formule générale (I) dans laquelle $R_1$ et $R_5$ représentent un atome d'hydrogène, $R_2$ et $R_4$ représentent un radical méthyle et $R_3$ représente un atome d'hydrogène ou un radical méthyl-3 butène-2 yle ou diméthyl-3,7 octadiène-2,6 yle.

Les produits de formule générale (I) sont des intermédiaires particulièrement intéressants en synthèse organique. Plus particulièrement, le procédé selon la présente invention permet d'accéder à la pseudo-ionone et à ses isomères qui sont utiles pour préparer les vitamines A et E, le ß-carotène ou le squalane, à la farnésylacétone qui permet de préparer la vitamine E par l'intermédiaire de la phytone, ou aux cyclanones qui conduisent à des produits utilisables en parfumerie.

Le réarrangement d'un alcool acétylénique de formule générale (II) en un produit de formule générale (I) est une réaction connue appelée transposition d'oxy-Cope. La transposition d'oxy-Cope a fait l'objet de nombreux travaux mais, compte tenu des conditions utilisées pour sa mise en œuvre, son intérêt pratique a été très limité. Plus particulièrement, le réarrangement thermique d'alcools acétyléniques permettant d'accéder à des composés de nature terpénique a été étudié par T. Onishi et coll., Synthesis, 651-654 (1980). L'inconvénient majeur du réarrangement thermique réside dans la fragilité de l'énol allénique, qui est le produit primaire de la réaction, et dans l'obtention de réactions secondaires parasites (réaction ène et rétro-ène). Le réarrangement thermique peut être amélioré en opérant en présence de solvants convenables tels que la N-méthylpyrrolidone, l'hexaméthylphosphotriamide ou le diméthylsulfoxyde, mais la sélectivité n'est pas satisfaisante.

Le réarrangement thermique d'alcools propargyliques a été utilisé pour la préparation de ionones ou d'irones (brevet français 7 815 136 publié sous le numéro 2 391 893) ou de cétones insaturées (brevet français 7 703 951 publié sous le numéro 2 340 923).

La demande de brevet européen EP 0 069 010 concerne le réarrangement d'oxy-Cope d'hexadiène-1,5 ols-3 en composés carbonylés δ-éthyléniques en présence d'une quantité catalytique d'un composé du palladium bivalent.

Le brevet français FR 1 576 228 concerne la préparation d'aldéhydes α, ω-diéthyléniques par transposition d'alcools acétyléniques ω-éthyléniques en présence d'un catalyseur choisi parmi les sels d'argent, de cuivre et d'or en milieu acide. Cependant, ce procédé ne constitue pas une réaction d'oxy-Cope.

Il a maintenant été trouvé, et c'est ce qui fait l'objet de la présente invention, que la transposition d'oxy-Cope des alcools acétyléniques de formule générale (II) peut être réalisée sélectivement en opérant à une température inférieure à 100 °C en présence d'un catalyseur convenable.

Les catalyseurs qui conviennent à la mise en œuvre du procédé selon l'invention sont choisis parmi les dérivés des métaux du groupe Ib de la classification périodique. Plus spécialement, les métaux dont les dérivés conviennent particulièrement bien sont le cuivre, l'argent et l'or. Peuvent être cités parmi ces dérivés le chlorure cuivreux, le bromure cuivreux, le chlorure cuivrique, l'acétate cuivreux, le trifluorométhane sulfonate d'argent, le nitrate d'argent, le borofluorure d'argent, l'acétate d'argent et le chlorure d'or.

Généralement, le catalyseur est mis en œuvre à raison de 0,001 à 1 mole par mole d'alcool acétylénique.

Pour améliorer la réactivité du catalyseur et la sélectivité de la réaction en éliminant les réactions secondaires de polymérisation, il est avantageux d'opérer en présence d'un sel de métal alcalin ou d'un sel d'ammonium quaternaire. Parmi les sels qui conviennent particulièrement bien peuvent être cités les halogénures (chlorures, fluorures), nitrates, trifluoracétates de sodium, potassium, lithium ou césium ou le chlorure de tétrabutylammonium.

La transposition d'oxy-Cope peut éventuellement être mise en œuvre en présence d'un solvant ou d'un mélange de solvants tels qu'une cétone (acétone), un alcool (éthanol), un éther (oxyde d'isopropyle), un solvant chloré (chlorobenzène), un nitrile (acétonitrile), un hydrocarbure aliphatique (hexane) ou un mélange tétrahydrofuranne-eau ou chlorure de méthylène-diméthylformamide.

Généralement, la durée de la réaction est comprise entre 15 minutes et 50 heures, la température étant comprise entre 0 et 100 °C.

La cétone diéthylénique de formule générale (I) est généralement isolée selon les méthodes habituelles et éventuellement après traitement du mélange réactionnel par une solution aqueuse basique, telle qu'une solution saturée de bicarbonate de sodium. Elle peut être purifiée par application des méthodes physiques telles que la distillation.

Le procédé selon la présente invention permet d'obtenir les cétones α, δ-diéthyléniques avec de bons rendements qui peuvent cependant varier selon la nature de l'alcool acétylénique mis en œuvre. Par ailleurs, le procédé est stéréosélectif et il conduit généralement à une proportion prépondérante de l'un des isomères.

Par ailleurs, il est possible d'utiliser les cétones α, δ-diéthyléniques obtenues selon le procédé de la présente invention sans isolement et purification préalables. Par exemple, le produit brut de la réaction peut être hydrogéné catalytiquement en cétone saturée correspondante avec des rendements généralement supérieurs à 80 % par rapport à l'alcool acétylénique mis en œuvre.

Les exemples suivants, donnés à titre non limitatif, montrent comment l'invention peut être mise en pratique.

### Exemple 1

On maintient à une température voisine de 40 °C et à l'abri de la lumière 0,192 g ($10^{-3}$ mole) d'isopropényl-4 diméthyl-3,7 octène-6 yne-1 ol-3 en présence de 0,257 g ($10^{-3}$ mole) de trifluorométhanesulfonate d'argent dans 10 $cm^3$ d'un mélange tétrahydrofuranne-eau (3-1 en volumes). Après 20 heures, le mélange réactionnel est dilué par 20 $cm^3$ d'éther éthylique et traité par 5 $cm^3$ d'une solution saturée de bicarbonate de sodium. La phase organique décantée est lavée à l'eau permutée jusqu'à neutralité puis séchée sur sulfate de magnésium. Après filtration, évaporation du solvant sous pression réduite (20 mm de mercure ; 2,7 kPa) et purification par chromatographie sur alumine, on obtient, avec un rendement de 57 %, 0,110 g de diméthyl-6,10 undécatriène-3,6,8 one-2 dont les caractéristiques sont les suivantes :

— spectre infra-rouge (à partir d'un film liquide) : bandes caractéristiques à 3 080, 3 030, 1 670, 1 625, 1 360, 1 250 et 980 $cm^{-1}$

— spectre de RMN ($CDCl_3$ ; δ en ppm, J en Hz) : 1,57 (s, 6H) ; 1,63 (s, 3H) ; 2,19 (s, 3H) ; 2,64 (t, 2H, J = 7) ; 2,80 (d, 2H, J = 7) ; 5,02 (t pert., 1H, J = 7) ; 5,12 (t pert., 1H, J = 7) ; 6,00 (d, 1H, J = 15) ; 6,70 (d × t, 1H, $J_1$ = 15, $J_2$ = 7).

La diméthyl-6,10 undécatriène-3,6,8 one-2 peut être obtenue en opérant comme dans l'exemple 1 mais en faisant varier la nature du catalyseur et éventuellement du co-catalyseur, le solvant, la température et la durée de réaction.

Les résultats sont rassemblés dans le tableau suivant :

(Voir Tableau pages 4, 5, 6)

| Exemples | Catalyseur | %(en mole) par rapport à l'alcool | Co-catalyseur | %(en mole) par rapport à l'alcool | Solvant | Température (°C) | Durée (heures) | Taux de transformation | Rendement (par rapport au produit transformé) |
|---|---|---|---|---|---|---|---|---|---|
| 2 | $AgOSO_2CF_3$ | 10 | - | - | acétone | 20 | 14 | 32 | 75 |
| 3 | $AgNO_3$ | 10 | - | - | acétone | 40 | 8 | 45 | 33 |
| 4 | $AgBF_4$ | 10 | - | - | THF-eau (2-1 en vol.) | 66 | 40 | 55 | 43 |
| 5 | $AgBF_4$ | 23 | | | $CH_2Cl_2$-DMF(2-1 en vol.) | 50 | 4 | 95 | 67 |
| 6 | $AgNO_3$ | 10 | K $NO_3$ | 80 | THF-eau (2-1 en vol.) | 66 | 24 | 98 | 40 |
| 7 | $AgNO_3$ | 10 | Cs $NO_3$ | 80 | THF-eau (2-1 en vol.) | 66 | 50 | 98 | 35 |
| 8 | $AgNO_3$ | 10 | K $NO_3$ | 20 | THF-eau (2-1 en vol.) | 66 | 15 | 94 | 45 |
| 9 | $AgNO_3$ | 10 | K $NO_3$ | 110 | THF-eau (2-1 en vol.) | 66 | 13 | 61 | 64 |
| 10 | $AgNO_3$ | 10 | K $NO_3$ | 300 | THF-eau (2-1 en vol.) | 66 | 13 | 60 | 66 |

| Exemples | Catalyseur | %(en mole) par rapport à l'alcool | Co-catalyseur | %(en mole) par rapport à l'alcool | Solvant | Température (°C) | Durée (heures) | Taux de transformation | Rendement (par rapport au produit transformé) |
|---|---|---|---|---|---|---|---|---|---|
| 11 | $AgNO_3$ | 20 | K $NO_3$ | 100 | THF-eau (2-1 en vol.) | 66 | 73 | 100 | 30 |
| 12 | $AgNO_3$ | 20 | K $NO_3$ | 100 | THF-eau (2-1 en vol.) | 66 | 15 | 100 | 52 |
| 13 | $AgOCOCF_3$ | 9 | | | THF-eau (1-1 en vol.) | 20 | 48 | 85 | 34 |
| 14 | $AgOCOCF_3$ | 21 | $LiOCOCF_3$ | 68 | THF-eau (1-1 en vol.) | 40 | 7,5 | 95 | 65 |
| 15 | $CuOCOCH_3$ | 20 | | | $CH_2Cl_2$-DMF (2-1 en vol.) | 50 | 4 | 63 | 75 |
| 16 | $CuCl_2$ | 21 | | | $CH_2Cl_2$-DMF(2-1 en vol.) | 50 | 4 | 35 | 55 |
| 17 | CuCl | 20 | | | $CH_2Cl_2$-DMF(2-1 en vol.) | 50 | 4 | 91 | 81 |
| 18 | CuBr | 20 | | | $CH_2Cl_2$-DMF(2-1 en vol.) | 50 | 4 | 18 | 58 |

| Exemples | Catalyseur | %(en mole) par rapport à l'alcool | Co-catalyseur | %(en mole) par rapport à l'alcool | Solvant | Température (°C) | Durée (heures) | Taux de transformation | Rendement (par rapport au produit transformé) |
|---|---|---|---|---|---|---|---|---|---|
| 19 | CuCl | 20 | | | isopropyléther | 60 | 6 | 17 | 52 |
| 20 | CuCl | 20 | | | THF-eau (1-1 en vol.) | 60 | 6 | 24 | 34 |
| 21 | CuCl | 20 | | | éthanol | 60 | 6 | 25 | 57 |
| 22 | CuCl | 20 | | | $C_6H_5Cl$ | 60 | 6 | 32 | 79 |
| 23 | CuCl | 20 | | | hexane | 60 | 6 | 55 | 71 |
| 24 | CuCl | 20 | | | acétonitrile | 60 | 6 | 76 | 80 |
| 25 | CuCl | 20 | | | | 60 | 6 | 94 | 80 |
| 26 | CuCl | 20 | | | | 100 | 0,25 | 88 | 89 |
| 27 | CuCl | 20 | LiCl | 45 | | 100 | 0,25 | 99 | 91 |
| 28 | CuCl | 5,7 | LiCl | 6,1 | | 100 | 0,5 | 77 | 91 |
| 29 | CuCl | 4,8 | NaCl | 6,4 | | 100 | 0,5 | 89 | 81 |
| 30 | CuCl | 5,7 | KCl | 7 | | 100 | 0,5 | 95 | 87 |
| 31 | CuCl | 20 | $(C_4H_9)_4\overset{+}{N}Cl^-$ | 19 | | 60 | 13 | 69 | 78 |
| 32 | CuCl | 20 | $LiOCOCF_3$ | 24 | | 60 | 4 | 98 | 91 |
| 33 | CuCl | 20 | $NaOCOCH_3$ | 31 | | 56 | 4 | 76 | 83 |
| 34 | CuCl | 21 | NaF | 54 | | 60 | 2 | 83 | 92 |
| 35 | CuCl | 2 | KCl | 3,3 | | 100 | 2 | 94 | 90 |

## Exemple 36

On maintient sous agitation à une température de 60 °C pendant 6 heures un mélange de 0,492 8 g ($2,56 \times 10^{-3}$ mole) de diméthyl-3,7 isopropényl-4 octène-6 yne-1 ol-3, de 0,088 3 g ($2,6 \times 10^{-4}$ mole) de chlorure aurique ($AuCl_3$, $2H_2O$) et 0,021 1 g ($2,8 \times 10^{-4}$ mole) de chlorure de potassium. Le mélange réactionnel est ensuite filtré sur une colonne contenant 2 g de silice en éluant avec de l'éther éthylique. Après évaporation de l'éluant sous pression réduite, on obtient 0,443 g d'un mélange contenant essentiellement la diméthyl-6,10 undécatriène-3,5,9 one-2 (pseudoionone), la diméthyl-6,10 undécatriène-3,6,9 one-2 et environ 1,5 % de l'alcool de départ.

Le mélange obtenu est hydrogéné en présence de 10 % en poids de palladium sur noir à 5 % dans l'éthanol. Après filtration et évaporation du solvant, on obtient exclusivement la diméthyl-6,10 undécanone-2.

## Exemple 37

On maintient, au reflux du tétrahydrofuranne et à l'abri de la lumière, 0,260 g ($10^{-3}$ mole) d'isopropényl-4 triméthyl-3,7,11 dodécadiène-6,10 yne-1 ol-3 en présence de 0,017 g ($10^{-4}$ mole) de nitrate d'argent et de 0,101 g ($10^{-3}$ mole) de nitrate de potassium dans 6 $cm^3$ d'un mélange tétrahydrofuranne-eau (2-1 en volumes). Après 15 heures de chauffage au reflux, le mélange réactionnel est traité dans les conditions décrites dans l'exemple 1. On obtient ainsi 0,120 g de triméthyl-6,10,14 pentadécatétraène-3,6,9,13 one-2 dont les caractéristiques sont les suivantes :

— spectre infra-rouge (à partir d'un film liquide) : bandes caractéristiques à 3 030, 1 680, 1 630, 1 255 et 985 $cm^{-1}$

— spectre de RMN ($CDCl_3$ ; δ en ppm ; J en Hz) : 1,55-1,72 (M, 12H) ; 1,88-2,12 (m, 4H) ; 2,15 (s, 3H) ; 2,70 (t, 2H, J = 7) ; 2,85 (d, 2H, J = 7) ; 4,9-5,25 (m, 3H) ; 5,95 (d, 1H, J = 17) ; 6,62 (d × t, 1H, $J_1$ = 17, $J_2$ = 7).

Le taux de transformation est de 80 % et le rendement par rapport à l'alcool transformé est de 57 %.

La triméthyl-6,10,14 pentadécatétraène-3,6,9,13 one-2 peut être obtenue en opérant comme dans l'exemple 38 mais en faisant varier la nature du catalyseur et éventuellement du co-catalyseur, le solvant, la température et la durée de la réaction. Les résultats sont rassemblés dans le tableau suivant :

(Voir Tableau page 7)

## Exemple 41

On maintient sous agitation, à une température de 100 °C pendant 1 heure un mélange de 0,510 g ($1,96 \times 10^{-3}$ mole) d'isopropényl-4 triméthyl-3,7,11 dodécadiène-6,10, yne-1 ol-3, de 0,011 8 g ($1,19 \times 10^{-4}$ mole) de chlorure cuivreux et de 0,0142 g ($1,9 \times 10^{-4}$ mole) de chlorure de potassium. Après filtration du mélange réactionnel sur une colonne de silice en éluant à l'éther éthylique puis évaporation de l'éluant, on obtient un liquide huileux jaunâtre contenant, d'après la chromatographie en phase vapeur, 6 % de l'alcool de départ et 81,6 % de triméthyl-6,10,14 pentadécatétraène-3,6,9,13 one-2 et de la triméthyl-6,10,14 pentadécatétraène-3,5,9,13 one-2 isomère.

L'hydrogénation de ce mélange dans les conditions décrites dans l'exemple 37 conduit exclusivement à la triméthyl-6,10,14 pentadécanone-2 (phytone) qui se présente sous la forme d'un mélange équimoléculaire des deux diastéréoisomères possibles d'après le spectre de RMN du $^{13}C$.

## Exemple 42

On maintient à une température voisine de 40 °C et à l'abri de la lumière, 0,180 g ($10^{-3}$ mole) d'isobuténуl-3 octyne-1 ol-3 en présence de 0,257 g ($10^{-3}$ mole) de trifluorométhanesulfonate d'argent dans 10 $cm^3$ d'un mélange tétrahydrofuranne-eau (3-1 en volumes). Après 7 heures de réaction, le mélange réactionnel est traité dans les conditions décrites dans l'exemple 1. On obtient 0,132 g de méthyl-2 undécadiène-1,4 one-6 dont les caractéristiques sont les suivantes :

— spectre infra-rouge (à partir d'un film liquide) : bandes caractéristiques à 3 080, 3 040, 1 680, 1 635, 985 et 890 $cm^{-1}$

— spectre de RMN ($CDCl_3$ ; δ en ppm, J en Hz) : 0,83 (t, 3H, J = 8) ; 1,24 (M, 4H) ; 1,55 (m, 2H) ; 1,67 (s, 3H) ; 2,47 (t, 2H, J = 11) ; 2,83 (d, 2H, J = 10) ; 4,68 (s élargi, 1H) ; 4,77 (s élargi, 1H) ; 6,08 (d, 1H, J = 22) ; 6,75 (d × t, 1H, $J_1$ = 22, $J_2$ = 10).

Le taux de transformation est de 100 % et le rendement par rapport au produit transformé est de 73 %.

## Exemple 43

On maintient, au reflux du tétrahydrofuranne et à l'abri de la lumière, 0,180 g ($10^{-3}$ mole) d'isobutényl-3 octyne-1 ol-3 en présence de 0,017 g ($10^{-4}$ mole) de nitrate d'argent et de 0,101 g ($10^{-3}$ mole) de nitrate de potassium dans 6 $cm^3$ d'un mélange tétrahydrofuranne-eau (2-1 en volumes). Après 6 heures de réaction on ajoute à nouveau 0;017 g de nitrate d'argent. Le reflux est maintenu

| Exemples | Catalyseur | %(en mole) par rapport à l'alcool | Co-catalyseur | %(en mole) par rapport à l'alcool | Solvant | Température (°C) | Durée (heures) | Taux de transformation | Rendement (par rapport au produit transformé) |
|---|---|---|---|---|---|---|---|---|---|
| 38 | $AgOSO_2CF_3$ | 100 | | | THF-eau (3-1 en vol.) | 20 | 48 | 88 | 62 |
| 39 | $AgNO_3$ | 100 | | | THF-eau (5-1 en vol.) | 20 | 16 | 89 | 58 |
| 40 | $AgNO_3$ | 10 | $K NO_3$ | 110 | THF-eau (2-1 en vol.) | 66 | 13 | 66 | 70 |

pendant encore 8,5 heures puis le mélange réactionnel est traité dans les conditions décrites dans l'exemple 1. On obtient 0,104 g de méthyl-2 undécadiène-1,4 one-6.

Le taux de transformation est de 90 % et le rendement par rapport au produit transformé est de 64 %.

## Exemple 44

On maintient, au reflux du tétrahydrofuranne et à l'abri de la lumière, 0,110 g ($10^{-3}$ mole) de méthyl-5 hexène-5 yne-1 ol-3 en présence de 0,257 g ($10^{-3}$ mole) de trifluorométhane-sulfonate d'argent dans 10 $cm^3$ d'un mélange tétrahydrofuranne-eau (3-1 en volumes). Après 1 heure de réaction, le mélange réactionnel est traité dans les conditions décrites dans l'exemple 1. On obtient 0,060 g de méthyl-5 hexadiène-2,5 al dont les caractéristiques sont les suivantes :

— spectre infra-rouge (à partir d'un film liquide) : bandes caractéristiques à 3 070, 3 020, 2 810, 2 730, 1 690, 1 650, 1 125, 970 et 895 $cm^{-1}$

— spectre de RMN ($CDCl_3$ ; δ en ppm, J en Hz) : 1,78 (s, 3H) ; 3,02 (d, 2H, J = 7) ; 4,75-4,95 (mt, 2H) ; 6,15 (d × d pert., 1H, $J_1$ = 15, $J_2$ = 8) ; 6,87 (d × t, 1H, $J_1$ = 15, $J_2$ = 7) ; 9,56 (d, 1H, J = 8).

Le taux de transformation est de 100 % et le rendement par rapport au produit transformé est de 55 %.

## Exemple 45

On maintient, au reflux du tétrahydrofuranne et à l'abri de la lumière, 0,110 g ($10^{-3}$ mole) de méthyl-5 hexène-5 yne-1 ol-3 en présence de 0,017 g ($10^{-4}$ mole) de nitrate d'argent et de 0,101 g ($10^{-3}$ mole) de nitrate de potassium dans 6 $cm^3$ d'un mélange tétrahydrofuranne-eau (2-1 en volumes). Après 5 heures de réaction on ajoute à nouveau 0,017 g ($10^{-4}$ mole) de nitrate d'argent puis on poursuit le chauffage pendant 1 heure. Le mélange réactionnel est traité dans les conditions décrites dans l'exemple 1. On obtient ainsi 0,055 g de méthyl-5 hexadiène-2,5 al.

Le taux de transformation est de 100 % et le rendement par rapport au produit transformé est de 50 %.

## Exemple 46

On maintient, au reflux du tétrahydrofurane et à l'abri de la lumière, le diméthyl-4,8 isopropényl-5 nonène-7 yne-2 ol-4 en présence d'une quantité équimoléculaire de trifluorométhane-sulfonate d'argent dans un mélange tétrahydrofurane-eau (3-1 en volumes). Après 21,5 heures de réaction, le mélange réactionnel est traité dans les conditions décrites dans l'exemple 1. On obtient ainsi la triméthyl-4,6,10 undécatriène-3,6,9 one-2 avec un rendement de 30 %.

## Exemple 47

On maintient à une température voisine de 60 °C et à l'abri de la lumière 0,025 g ($10^{-4}$ mole) d'isopropényl-2 yne-1 cyclododécanol en présence de 0,030 g de trifluorométhanesulfonate d'argent dans 1,2 $cm^3$ d'un mélange tétrahydrofuranne-eau (5-1 en volume). Après 24 heures de réaction, le mélange réactionnel est traité dans les conditions décrites dans l'exemple 1. On obtient ainsi 0,010 g de méthyl-5 cyclohexadécadiène-2,5 one dont les caractéristiques sont les suivantes :

— m/e = 248

— spectre infra-rouge (à partir d'un film liquide) : bandes caractéristiques à 1 695, 1 670 et 1 623 $cm^{-1}$

— spectre de RMN du proton à 360 MHz ($CDCl_3$ ; δ en ppm ; J en Hz ; référence interne hexaméthyldisilane) : 1,22 (m, 14H) ; 1,55 (m, 2H) ; 1,66 (s, 3H) ; 1,92 (m, 2H) ; 2,41 (t, 2H) ; 2,87 (dd, $J_1$ voisin de 6,5, $J_2$ voisin de 2, 2H) ; 5,23 (t, J voisin de 7, 1H) ; 6,04 (dt, $J_1$ voisin de 15, $J_2$ voisin de 1,5, 1H) ; 6,72 (dt, $J_1$ voisin de 15, $J_2$ voisin de 6,5, 1H).

## Exemple 48

On maintient à une température voisine de 40 °C et à l'abri de la lumière 0,960 g ($5.10^{-3}$ mole) d'isopropényl-4 diméthyl-3,7, octène-6 yne-1 ol-3 en présence de 1,285 g ($5.10^{-3}$ mole) de trifluorométhanesulfonate d'argent dans 50 $cm^3$ d'un mélange tétrahydrofuranne-eau (3-1 en volume). Après 20 heures de réaction et traitement du mélange réactionnel dans les conditions de l'exemple 1, le produit brut obtenu est hydrogéné en présence de palladium sur noir à 5 % dans l'hexane. Après une chromatographie sur colonne de silice sous faible pression en éluant avec un mélange éther-éther de pétrole (3-7 en volume), on obtient 0,792 g de diméthyl-6,10 undécanone-2 dont les caractéristiques sont les suivantes :

— spectre infra-rouge (à partir d'un film liquide) ; bandes caractéristiques à : 2 950, 2 920, 2 860, 1 715, 1 460, 1 360, 1 160

— spectre RMN ($CDCl_3$, δ en ppm, J en Hz) ; 0,88 (d, J = 6,5, 9H) ; 1,1-1,8 (M, 12H) ; 2,12 (s. 3H) ; 2,4 (t, J = 7, 2H)

— masse 198 ($M^+$) (25 %), 170 (12 %), 137 (12 %), 110 (12 %), 100 (100 %), 85 (95 %).
Le rendement est de 80 % par rapport à l'alcool acétylénique mis en œuvre.

**Revendications**

1. Procédé de préparation de composés carbonylés α,δ-diéthyléniques de formule générale :

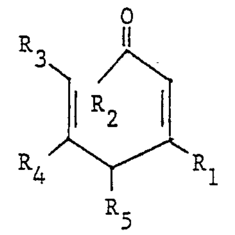

dans laquelle $R_1$, $R_2$, $R_3$, $R_4$ et $R_4$, identiques ou différents, représentent un atome d'hydrogène ou un radical hydrocarboné, $R_5$ représente un atome d'hydrogène, étant entendu que $R_2$ et $R_3$ peuvent former ensemble un radical alcoylène $(—CH_2—)_n$ dont un ou plusieurs atomes de carbone peuvent être éventuellement substitués par un ou plusieurs radicaux alcoyles contenant 1 à 4 atomes de carbone et dans lequel n représente un nombre entier compris entre 3 et 20 inclusivement et que $R_2$ et $R_5$ peuvent former ensemble un cycle contenant de 6 à 16 chaînons, caractérisé en ce que l'on effectue la transposition d'un alcool acétylénique de formule générale :

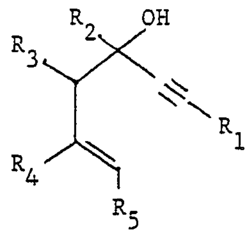

dans laquelle $R_1$, $R_2$, $R_3$ et $R_5$ sont définis comme précédemment à une température inférieure à 100 °C en présence d'un catalyseur choisi parmi les dérivés des métaux du groupe Ib de la classification périodique.

2. Procédé selon la revendication 1 caractérisé en ce que le catalyseur est choisi parmi les dérivés du cuivre, de l'argent et de l'or.

3. Procédé selon la revendication 1 caractérisé en ce que le catalyseur est choisi parmi le chlorure cuivreux, le bromure cuivreux, le chlorure cuivrique, l'acétate cuivreux, le trifluorométhanesulfonate d'argent, le nitrate d'argent, le borofluorure d'argent, l'acétate d'argent et de chlorure d'or.

4. Procédé selon la revendication 1 caractérisé en ce que le catalyseur est mis en œuvre à raison 0,001 à 1 mole par mole d'alcool acétylénique mis en œuvre.

5. Procédé selon la revendication 1 caractérisé en ce que l'on opère en présence d'un sel de métal alcalin ou d'un sel d'ammonium quaternaire.

6. Procédé selon la revendication 5 caractérisé en ce que le sel est choisi parmi les halogénures, les nitrates, trifluoroacétates de sodium, potassium, lithium ou césium et le chlorure de tétrabutylammonium.

7. Procédé selon la revendication 1 caractérisé en ce que l'on opère dans un solvant organique choisi parmi les cétones, les alcools, les éthers, les nitriles, les amides, les solvants chlorés, les hydrocarbures aliphatiques ou leurs mélanges ou dans un mélange hydroorganique.

8. Procédé selon la revendication 1 caractérisé en ce que l'on met en œuvre un alcool acéthylénique de formule générale :

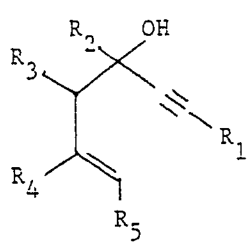

dans laquelle $R_1$, $R_2$, $R_3$ et $R_4$, identiques ou différents, représentent un atome d'hydrogène ou un radical acyclique contenant 1 à 20 atomes de carbone dont la chaîne peut contenir une ou plusieurs doubles ou triples liaisons et $R_5$ représente un atome d'hydrogène, $R_2$ et $R_3$ pouvant en outre représenter un radical alcoylène $(-CH_2-)_n$ dont un ou plusieurs atomes de carbone peuvent être éventuellement substitués par un ou plusieurs radicaux alcoyles contenant 1 à 4 atomes de carbone et dans lequel n est compris entre 3 et 20 inclusivement.

9. Procédé selon la revendication 1 caractérisé en ce que l'on met en œuvre un alcool acétylénique de formule générale :

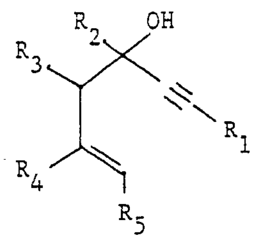

dans laquelle $R_1$ et $R_5$ représentent un atome d'hydrogène, $R_2$ et $R_4$ représentent un radical méthyle et $R_3$ représente un radical méthyl-3 butène-2 yle ou diméthyl-3,7 octadiène-2,6 yle.

## Claims

1. Process for preparing α,δ-diethylenic carbonyl compounds of general formula :

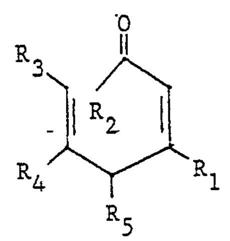

in which $R_1$, $R_2$, $R_3$ and $R_4$, which may be identical or different, denote a hydrogen atom or a hydrocarbon radical and $R_5$ denotes a hydrogen atom, with the proviso that $R_2$ and $R_3$ together can form an alkylene radical $(-CH_2-)_n$ in which one or more carbon atoms can optionally be substituted with one or more alkyl radicals containing 1 to 4 carbon atoms and in which n denotes an integer between 3 and 20 inclusive, and that $R_2$ and $R_5$ together can form a ring containing from 6 to 16 members, characterized in that the rearrangement of an acetylenic alcohol of general formula :

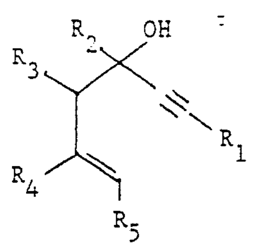

in which $R_1$, $R_2$, $R_3$, $R_4$ and $R_5$ are defined as above, is performed at a temperature below 100 °C in the presence of a catalyst chosen from derivatives of metals of Group Ib of the Periodic Classification.

2. Process according to Claim 1, characterized in that the catalyst is chosen from derivatives of copper, silver and gold.

3. Process according to Claim 1, characterized in that the catalyst is chosen from cuprous chloride, cuprous bromide, cupric chloride, cuprous acetate, silver trifluoromethane sulphonate, silver nitrate, silver borofluoride, silver acetate and gold chloride.

4. Process according to Claim 1, characterized in that the catalyst is employed in the proportion of 0.001 to 1 mole per mole of acetylenic alcohol employed.

5. Process according to Claim 1, characterized in that the reaction is performed in the presence of an

alkali metal salt or quaternary ammonium salt.

6. Process according to Claim 5, characterized in that the salt is chosen from sodium, potassium, lithium or caesium halides, nitrates or trifluoroacetates and tetrabutylammonium chloride.

7. Process according to Claim 1, characterized in that the reaction is performed in an organic solvent chosen from ketones, alcohols, ethers, nitriles, amides, chlorinated solvents and aliphatic hydrocarbons, or mixtures thereof, or in an aqueous organic mixture.

8. Process according to Claim 1, characterized in that there is employed an acetylenic alcohol of general formula :

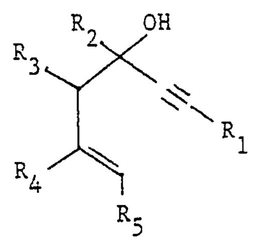

in which $R_1$, $R_2$, $R_3$ and $R_4$, which may be identical or different, denote a hydrogen atom or an acyclic radical containing 1 to 20 carbon atoms in which the chain can contain one or more double or triple bonds, and $R_5$ denotes a hydrogen atom, $R_2$ and $R_3$ being able, in addition, to denote an alkylene radical $(-CH_2-)_n$ in which one or more carbon atoms can optionally be substituted with one or more alkyl radicals containing 1 to 4 carbon atoms and in which n is between 3 and 20 inclusive.

9. Process according to Claim 1, characterized in that there is employed an acetylenic alcohol of general formula :

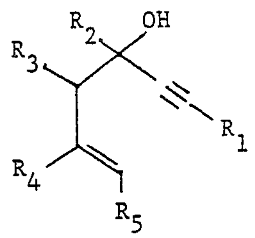

in which $R_1$ and $R_5$ denote a hydrogen atom, $R_2$ and $R_4$ denote a methyl radical and $R_3$ denotes a 3-methyl-2-butenyl or 3,7-dimethyl-2,6-octadienyl radical.

**Patentansprüche**

1. Verfahren zur Herstellung α,δ-diäthylenischer Carbonylverbindungen der allgemeinen Formel :

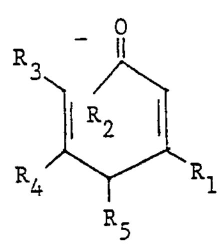

in whelcher $R_1$, $R_2$, $R_3$ und $R_4$ gleich oder voneinander verschieden sind und ein Wasserstoffatom oder einen Kohlenwasserstoffrest darstellen und $R_5$ ein Wasserstoffatom darstellt, wobei $R_2$ und $R_3$ zusammen einen Alkylenrest $(-CH_2-)_n$ bilden können, worin ein oder mehrere Kohlenstoffatome gegebenenfalls durch einen oder mehrere Alkylreste mit 1 bis 4 Kohlenstoffatomen substituiert sein können und worin n für eine ganze Zahl zwischen 3 und 20 inklusive steht, und wobei $R_2$ und $R_5$ zusammen einen Ring mit 6 bis 16 Gliedern bilden können, dadurch gekennzeichnet, daß man die Umlagerung eines Acetylenalkohols der allgemeinen Formel :

(Siehe Formel Seite 13 f.)

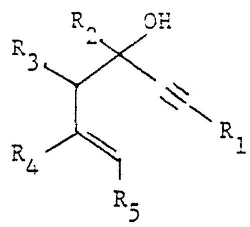

in welcher $R_1$, $R_2$, $R_3$, $R_4$ und $R_5$ die obige Bedeutung haben, bei einer Temperatur unterhalb 100 $cm^3$ in Gegenwart eines Katalysators, ausgewählt aus den Derivaten der Metalle der Gruppe Ib des Periodensystems, ausführt.

2. Verfahren nach Anspruch 1, dadurch gekennzeichnet, daß der Katalysator ausgewählt ist aus den Derivaten von Kupfer, Silber und Gold.

3. Verfahren nach Anspruch 1, dadurch gekennzeichnet, daß der Katalysator ausgewählt ist aus Kupfer(I)chlorid, Kupfer(I)bromid, Kupfer(II)chlorid, Kupfer(I)acetat, Silbertrifluormethansulfonat, Silbernitrat, Silberborfluorid, Silberacetat und Goldchlorid.

4. Verfahren nach Anspruch 1, dadurch gekennzeichnet, daß der Katalysator in einer Menge von 0,001 bis 1 Mol pro Mol eingesetztem Acetylenalkohol eingesetzt wird.

5. Verfahren nach Anspruch 1, dadurch gekennzeichnet, daß man in Gegenwart eines Alkalimetallsalzes oder eines quaternären Ammoniumsalzes arbeitet.

6. Verfahren nach Anspruch 5, dadurch gekennzeichnet, daß das Salz ausgewählt ist aus den Natrium-, Kalium-, Lithium- oder Cäsiumhalogeniden, -nitraten und Trifluoracetaten und Tetrabutylammoniumchlorid.

7. Verfahren nach Anspruch 1, dadurch gekennzeichnet, daß man in einem organischen Lösungsmittel, ausgewählt aus den Ketonen, den Alkoholen, den Äthern, den Nitrilen, den Amiden, den chlorierten Lösungsmitteln, den aliphatischen Kohlenwasserstoffen oder deren Mischungen oder in einer wässerig/organischen Mischung arbeitet.

8. Verfahren nach Anspruch 1, dadurch gekennzeichnet, daß man einen Acetylenalkohol der allgemeinen Formel

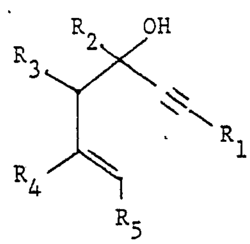

einsetzt, worin $R_1$, $R_2$, $R_3$ und $R_4$ gleich oder voneinander verschieden sind und ein Wasserstoffatom oder einen acyclischen Rest mit 1 bis 20 Kohlenstoffatomen darstellen, dessen Kette eine oder mehrere Doppel- oder Dreifachbindungen enthalten kann, und $R_5$ ein Wasserstoffatom darstellt, wobei $R_2$ und $R_3$ außerdem einen Alkylenrest $(-CH_2-)_n$ darstellen können, worin ein oder mehrere Kohlenstoffatome gegebenenfalls durch einen oder mehrere Alkylreste mit 1 bis 4 Kohlenstoffatomen substituiert sein können und worin n zwischen 3 und 20 inklusive liegt.

9. Verfahren nach Anspruch 1, dadurch gekennzeichnet, daß man einen Acetylenalkohol der allgemeinen Formel :

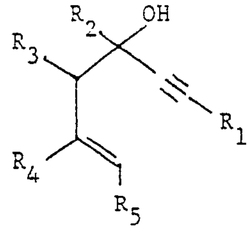

einsetzt, worin $R_1$ und $R_5$ ein Wasserstoffatom darstellen, $R_2$ und $R_4$ einen Methylrest darstellen und $R_3$ einen 3-Methyl-2-butenyl- oder 3,7-Dimethyl-2,6-octadienylrest darstellen.